(19) Europäisches Patentamt
European Patent Office
Office européen des brevets

(11) Numéro de publication: 0 091 385 B1

(12) FASCICULE DE BREVET EUROPEEN

(45) Date de publication du fascicule du brevet: 12.06.85

(51) Int. Cl.[4]: C 07 C 121/75, C 07 C 91/28, C 07 C 93/04, C 07 C 120/00

(21) Numéro de dépôt: 83420041.2

(22) Date de dépôt: 14.03.83

(54) **Procédé de préparation de p-hydroxybenzylnitriles ou p-hydroxybenzylamines.**

(30) Priorité: 15.03.82 US 358397

(43) Date de publication de la demande: 12.10.83 Bulletin 83/41

(45) Mention de la délivrance du brevet: 12.06.85 Bulletin 85/24

(84) Etats contractants désignés: AT BE CH DE FR GB IT LI LU NL SE

(56) Documents cités:
DE - A - 2 457 079
DE - C - 865 455

JOURNAL OF THE AMERICAN CHEMICAL SOCIETY, vol. 70, 1948, E.R. ALEXANDER et al. "A low pressure reductive alkylation method for the conversion of ketones to primary amines", pages 1315-1316
TETRAHEDRON, vol. 29, 1973, J.H. SHORT et al. "Synthesis of phenethylamines from phenylacetonitriles obtained by alkylation of cyanide ion with mannich bases from phenols and other benzylamines", pages 1931-1939
COMPT. RENDUS, 210 (1940), p. 403-405, CANTAREL, R.

(73) Titulaire: RHONE-POULENC SPECIALITES CHIMIQUES, "Les Miroirs" 18, Avenue d'Alsace, F-92400 Courbevoie (FR)

(72) Inventeur: Farber, Léon, Franklin Greens South, Somerset New Jersey 08873 (US)
Inventeur: Gradeff, Peter S., P.O. Box 278, Pottersville New Jersey 07979 (US)

(74) Mandataire: Rioufrays, Roger et al, RHONE-POULENC RECHERCHES Service Brevets Chimie et Polymères Centre de Recherches de Saint-Fons B.P. 62, F-69192 St-Fons Cedex (FR)

EP 0 091 385 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un procédé pour la préparation de p-hydroxybenzylnitriles.

On a préparé jusqu'à présent le p-hydroxybenzylnitrile en passant par des voies diverses dont aucune ne donne entière satisfaction et qui toutes se servent de matières de départ et/ou de réactifs qui sont relativement coûteux.

Selon le brevet américain N° 4154757, on fait réagir l'acide p-hydroxymandélique avec l'ion cyanure, d'une façon commode sous la forme d'un cyanure de métal alcalin comme le potassium ou le sodium, en solution dans un solvant aprotique dipolaire dont le point d'ébullition est relativement élevé, comme le N,N-diméthylformamide, à une température qui est comprise entre 120 et 190° C, facultativement en présence d'une base. Le produit de départ, l'acide p-hydroxymandélique, s'obtient sous la forme de p-hydroxymandélate de sodium ou de potassium monohydraté, en application de cette façon de procéder.

Le brevet américain N° 4154758 décrit un procédé consistant à faire réagir l'acide p-hydroxyphénylaminoacétique avec l'ion cyanure, d'une façon commode sous la forme d'un cyanure de métal alcalin tel que le cyanure de sodium ou de potassium, en solution dans un solvant aprotique dipolaire dont le point d'ébullition est relativement élevé, comme le N,N-diméthylformamide, à température comprise entre 100 et 250° C.

Bien que ces façons de procéder fournissent de bons rendements, chacune des matières de départ est coûteuse et aucune n'est facilement disponible sur le marché en grandes quantités; il convient en outre de les préparer spécialement.

Le brevet américain N° 3983160 et la demande de brevet allemand N° 2457079 décrivent la préparation d'un p-hydroxyphénylacétonitrile de formule:

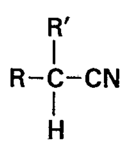

dans laquelle:

— R représente un radical ortho- ou parahydroxyphényle qui, de plus, peut être encore substitué par un ou deux substituants, indépendamment choisis parmi le groupe constitué par un radical alcoyle inférieur ou alcoxy inférieur ou bien n'être pas substitué, et

— R' représente un atome d'hydrogène, un radical alcoyle inférieur, phényle ou carbo(alcoxy inférieur),

préparation qui consiste à laisser réagir l'alcool correspondant ortho- ou parahydroxybenzylique de formule:

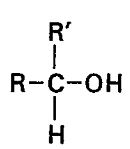

avec de l'acide cyanhydrique en présence d'un diluant à une température comprise entre environ 80 et environ 190° C.

Une autre façon de procéder décrite par Short *et al.*, «Tetrahedron», *29*, 1931 (1973), pour la préparation des benzylamines s'effectue par la réaction de Mannich sur les phénols correspondants. Short *et al.* ont également décrit la réaction de la vanillylamine avec le cyanure de potassium pour former le cyanure de benzyle correspondant, conformément à la réaction représentée par le schéma ci-après:

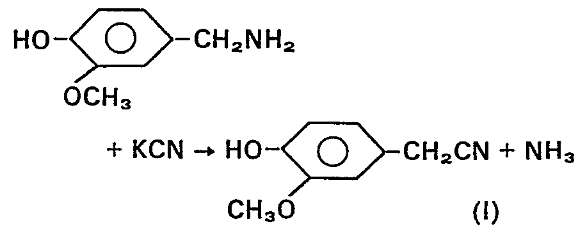

(I)

La préparation des phénylacétonitriles en partant de benzylamines se fait par dissolution de l'amine dans du diméthylformamide puis par addition du cyanure de potassium; le milieu réactionnel est chauffé sous agitation à 110-130° C pendant 6 h;

Hakelsberg, «J. Am. Chem. Soc.», *70* 2811 (1948), décrit l'amination réductrice des cétones en amines primaires par une solution éthanolique d'ammoniac à pression atmosphérique d'hydrogène et en présence de nickel de Raney. Dans la plupart des cas, on obtient également une amine secondaire comme sous-produit.

Emerson, «Organic Reactions», vol. 4, p. 174 (1948), note que le benzaldéhyde est transformé en benzylamine avec de bons rendements par hydrogénation en présence d'une quantité équimoléculaire d'ammoniac dans l'éthanol et de nickel de Raney, sous une pression de 20 à 150 atm. Une petite quantité de dibenzylamine est également formée qui peut être aisément séparée en raison de la différence des points d'ébullition des deux produits. Cependant, on indique que peu d'autres aldéhydes aromatiques ont été transformés en amines primaires par amination réductrice. Le tolualdéhyde conduit à l'amine primaire avec de bons rendements de même que l'orthochlorobenzaldéhyde [cf. Winans, «J. Am. Chem. Soc.» *61* 3566 (1939)]. Les aldéhydes contenant d'autres noyaux aromatiques n'ont apparemment pas été soumis à ce type de réaction.

Emerson indique que l'hydrogénation d'une solution éthanolique d'ammoniaque et de l'aldéhyde en présence de nickel de Raney est le procédé le plus efficace, l'ammoniaque étant utilisée en excès pour minimiser la formation d'amine secondaire. Les meilleurs résultats sont atteints sous des pressions d'hydrogène de 20 à 150 atm. A une pression inférieure à 20 atm, l'hydrogénation est trop lente. De plus, une température d'au moins 40° C est considérée comme nécessaire pour que la réaction démarre, et de bons résultats ont été obtenus à des températures de 40 à 150° C.

Alexander *et al.*, «J. Am. Chem. Soc.», *70* 1315 (1948), décrivent un procédé d'amination réductrice des cétones en amines primaires à basse pression. Ils indiquent qu'il est connu que les composés carbonyles sont hydrogénés, en présence d'ammoniac, en un mélange d'amines primaires, secondaires et tertiaires. Les meilleurs rendements et les plus reproductibles sont obtenus sur nickel de Raney et sous des pressions d'hydrogène de 20 à 150 atm et à des températures de 40 à 150° C conformément à l'enseignement de Mignonac, «Compt. Rend.», *172* 223 (1921); Schwogler *et al.*, «J. Am. Chem. Soc.», *61* 3499 (1939); Winans, *loc. cit.*, Alexander *et al.*, *loc. cit.*, ont essayé d'améliorer le procédé basse pression de Mignonac en utilisant une solution méthanolique d'ammoniac et de l'oxyde de platine comme catalyseur. Outre diverses cétones, l'isobutyraldéhyde et le benzaldéhyde ont été soumis à l'amination réductrice en présence de chlorure d'ammonium. Les rendements obtenus ont été faibles, respectivement de 10 et de 15% en amine primaire, à la différence des cétones qui conduisent à des rendements très supérieurs allant jusqu'à 69% dans le cas de l'acétophénone. Winans lui-même, *loc. cit.*, indique que des pressions d'hydrogène aussi basses que 20 atm sont satisfaisantes, mias qu'on a recours en général à des pressions plus élevées allant jusqu'à 100 atm et à des températures de 40 à 75° C. L'ammoniac est utilisé classiquement sous forme de solutions alcooliques.

Dans le brevet japonais N° 17730, on a décrit la préparation de la vanillylamine par la réaction de la vanilline avec de l'ammoniac liquide en présence de nickel de Raney en tant que catalyseur entre 40 et 70° C sous une pression d'hydrogène de 100 atm en chauffant ce mélange pendant 6 h.

Selon le brevet canadien N° 1067099, on prépare des cyanures d'alcoyl(inférieur)-3 hydroxy-4 benzyle par réaction d'une N-phénylalcoxy(inférieur)-3 hydroxy-4 benzylamine de formule:

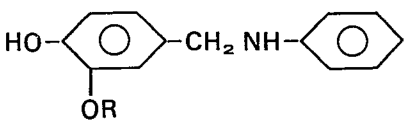
(II)

dans laquelle R représente un radical alcoyle inférieur, avec un cyanure de métal alcalin, dans un solvant polaire comme le diméthylformamide, entre 100 et 150° C. On prépare la N-phénylalcoxy(inférieur)-3 hydroxy-4 benzylamine par la suite de réactions:

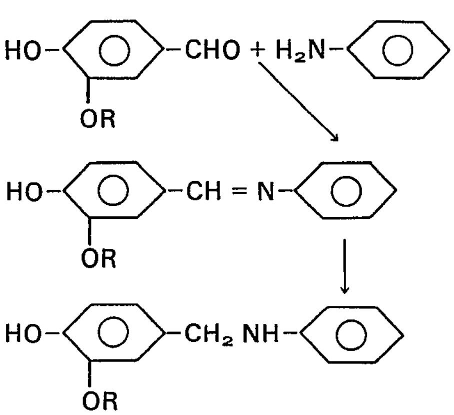
(III)

(IV)

(II)

On conduit la réduction à une température ambiante, faible, en se servant d'un hydrure métallique comme le borohydrure de sodium, qui est un produit de remplacement de l'hydrogène très coûteux.

La présente invention a pour objet un procédé de préparation de p-hydroxybenzylnitriles à partir du p-hydroxybenzaldehyde correspondant, en deux étapes, par:

a) réduction d'un benzaldéhyde de formule générale:

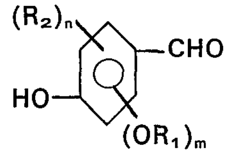

dans laquelle:
- $R_1$ reporésente un radical méthyle ou éthyle,
- $R_2$ représente un radical méthyle ou éthyle,
- m est un nombre compris entre 0 et 4,
- n est un nombre compris entre 0 et 4,

pour former la benzylamine correspondante de formule générale:

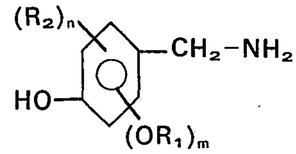

b) remplacement du groupe amino de la benzylamine par un groupe nitrile par réaction avec un cyanure choisi dans le groupe constitué par les cyanures des métaux alcalins et l'acide cyanhydrique, en solution dans un solvant choisi parmi le groupe constitué par le diméthylformamide, le diméthylsulfoxyde et la diméthylpyrrolidone, à une température comprise entre 80° C et la température de reflux de la solution pour former le benzylnitrile correspondant de formule:

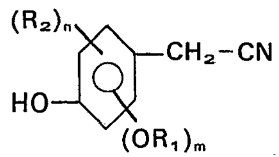

caractérisé en ce que, à l'étape a, la réduction se fait avec de l'hydrogène sous une pression comprise entre la pression atmosphérique et 10 bar, à une température comprise entre 20 et 100° C, dans un milieu réactionnel aqueux comportant de l'ammoniaque en une quantité comprise entre la quantité stœchiométrique et 10 fois la quantité stœchiométrique, et un catalyseur du type nickel de Raney.

Le cyanure de benzyle est ensuite récupéré aisément, par exemple par élimination du solvant par distillation.

La première phase de cette synthèse, qui est désignée par amination réductrice de l'hydroxybenzaldéhyde avec de l'ammoniaque en phase aqueuse sous basse pression, est nouvelle et elle s'applique non pas uniquement au p-hydroxybenzaldéhyde, qui n'avait encore jamais été transformé auparavant en p-hydroxybenzylamine par

amination réductrice, mais également aux o- et m-hydroxybenzaldéhydes. La seconde étape de la réaction ne procède qu'avec des p-hydroxybenzylamines. Par conséquent,·la synthèse en deux étapes est limitée à la réaction des p-hydroxybenzaldéhydes. Si toutefois la benzylamine n'a pas à être transformée en nitrile correspondant, on peut également appliquer la première phase du procédé aux o- et m-hydroxybenzaldéhydes.

La présente invention concerne donc également un procédé de préparation d'hydroxybenzylamines primaires qui consiste à réduire un benzaldéhyde présentant la formule:

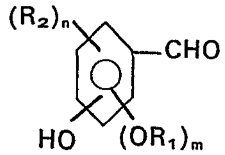

dans laquelle:

le groupe hydroxyle est en position ortho, méta ou para, $R_1$ représente un radical méthyle ou éthyle,

m est un nombre de 0 à 4

n est un nombre de 0 à 4,

avec de l'hydrogène gazeux sous une pression entre la pression atmosphérique et environ 10 bar à une température comprise entre environ 20 et environ 100° C, dans un milieu réactionnel aqueux comportant de l'ammoniaque en une quantité comprise entre environ la stœchiométrie et environ 10 fois la quantité stœchiométrique et du nickel de Raney à titre de catalyseur, pour former la benzylamine correspondante:

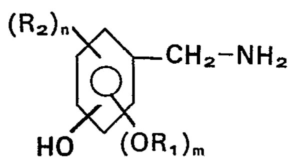

Le milieu réactionnel au cours de la première phase de la réaction est un système aqueux dans lequel l'aldéhyde en cours de réaction peut ou non passer complètement en solution dans la phase aqueuse au début de la réaction. Le produit de la réaction, la benzylamine correspondante, est insoluble dans le mélange réactionnel et, par conséquent, précipite à mesure que la réaction progresse.

A mesure qu'augmente la quantité du précipité de benzylamine, la viscosité du mélange réactionnel croît aussi. Pour permettre l'agitation de ce mélange, il peut être souhaitable d'ajouter une quantité supplémentaire d'eau, si nécessaire, afin de pouvoir abaisser la viscosité ou bien, en variante, un alcool aliphatique inférieur qui est miscible avec l'eau comme le méthanol ou l'éthanol à titre de diluant. Toutefois l'alcanol inférieur ne prend pas part à la réaction, mais ne sert qu'à abaisser la viscosité.

Alors que le procédé est applicable à un p-hydroxybenzaldéhyde quelconque, il est particulièrement intéressant industriellement de l'appliquer au p-hydroxybenzaldéhyde et à la vanilline.

D'une façon surprenante, la réduction ne requiert pas des pressions d'hydrogène élevées. La réaction se déroule très lentement à la pression atmosphérique. C'est par conséquent une raison d'appliquer une pression supérieure à celle de l'atmosphère; si on le désire, des pressions allant jusqu'à environ 10 bar peuvent être appliquées. La gamme préférée pour la pression d'hydrogène est comprise entre la pression atmosphérique et environ 5 bar.

Donc, il est possible de conduire la réaction dans un récipient clos, pressurisé avec la quantité d'hydrogène qui est requise pour l'amination réductrice, ce qui permet à la pression dans le récipient de parvenir au maximum, quel qu'il soit, pour la température choisie pour la réaction.

La réaction a lieu à la température ambiante et, par conséquent, il n'y a pas de raison d'appliquer une température supérieure, bien que la réaction se déroule plus rapidement aux températures élevées. Des températures allant jusqu'à 100° C environ peuvent être facilement appliquées. On peut appliquer des températures qui sont quelque peu plus élevées, si la pression d'hydrogène est maintenue bien au-dessus de la pression atmosphérique.

L'ammoniaque est évidemment nécessaire en une quantité au moins stœchiométrique pour former le groupe amino primaire $-NH_2$. On a trouvé qu'il était souhaitable d'en utiliser un excès afin d'assurer l'achèvement total de la réaction. On peut se servir d'un excès considérable, allant jusqu'à 10 mol environ, si on le désire, bien que cela ne soit pas nécessaire. Mais de préférence, toutefois, la quantité d'ammoniaque est comprise entre la quantité stœchiométrique, c'est-à-dire 1 mol par mole de benzaldéhyde et 5 mol.

La quantité de nickel de Raney utilisée comme catalyseur n'est pas critique. La réaction se produit avec des quantités qui sont aussi faibles que 1% en poids de mélange réactionnel. Bien qu'il soit possible d'utiliser des quantités supérieures, allant jusqu'à environ 25% du poids du mélange réactionnel, ces grandes quantités ne sont pas véritablement requises. La quantité préférée est comprise entre 5 et 10% du poids du mélange réactionnel.

La seconde étape de la réaction a été appliquée par J. H. Short *et al.*, «Tetrahedron», *29*, 134 (1973), à l'hydroxy-4 méthoxy-3 benzylamine et à ses dérivés N,N-méthylé et N,N-diméthylé qui conduisent à des rendements respectivement de 64, 58 et 56% en hydroxy-4 méthoxy-3 phénylacétonitrile.

Un groupe OH en position méta empêche le déroulement de cette réaction. On n'a pu isoler aucun nitrile correspondant lorsqu'on laisse l'hydroxy-3 méthoxy-4 benzylamine (isovanillylamine) réagir avec l'ion cyanure de la façon habituelle. Le seul produit qui a été isolé a été un produit de transamidation, le N-(hydroxy-3 méthoxy-1 benzyl)formamide.

La seconde phase de la réaction est directe et on la conduit au mieux en solution dans un solvant organique inerte; on préfère le diméthylformamide, le diméthylsulfoxyde et la diméthylpyrrolidone. La quantité de solvant n'est pas critique et

elle peut être comprise entre environ 300 et environ 2000 g/mol et de préférence entre environ 500 et environ 1200 g/mol d'hydroxybenzylamine; bien qu'il soit possible d'utiliser de plus grandes quantités de solvant.

La température réactionnelle est modérée puisqu'elle est comprise entre 80° C environ et la température de reflux du solvant utilisé. A la fin de la réaction, on chasse le solvant sous pression réduite et à basse température. On peut ensuite reprendre le benzylnitrile dans de l'eau acidifiée et l'on effectue l'extraction du mélange aqueux avec par exemple de la méthylisobutylcétone ou de l'éther isopropylique.

Les exemples qui suivent représentent des formes préférées de mise en œuvre de l'invention.

*Exemples 1 à 7*

On effectue sept réactions selon le mode opératoire suivant:

Dans un appareil d'hydrogénation de 500 ml agité, on introduit les quantités adéquates de p-hydroxybenzaldéhyde, d'ammoniaque, de méthanol et de nickel de Raney présentées pour chaque exemple du tableau I, puis on purge l'air du récipient par de l'hydrogène à la pression atmosphérique. On ferme le récipient et l'on conduit ensuite la réaction d'amination réductrice à la température qui est indiquée au tableau I, pendant la durée qui est indiquée. A l'achèvement de la réaction, pour les exemples 1, 3, 4, 5, 6, et 7, on détermine le rendement par analyse chromatographique en phase gazeuse. On obtient les résultats suivants:

*Tableau I*

| Exemples N° | Proportions molaires | | | RaNi (g) | Durée (h) | Température (° C) | Rendement (%) |
|---|---|---|---|---|---|---|---|
| | PHB* | $NH_4OH$ (aq.) (R.M./%$NH_3$) *** | $CH_3OH$ (ml) | | | | |
| 1 | 1 | 5,0/25,7 | 500 | 10 | 6,5 | ambiante | quantitatif |
| 2 | 1 | 2,0/25,7 | 500 | 10 | 9,5 | ambiante | non calculé |
| 3 | 1 | 5,0/25,7 | 250 | 10 | 7 | ambiante | 96,9 |
| 4 | 1 | 5,0/24,2 | 0 (500 ml $H_2O$) | 11,7 | 23 | ambiante | 98,3 |
| 5 | 1 | 5,0/25,7 | 250 | 10 | 7 | ambiante | 97,6 |
| 6 | 1 | 5,1/23,0 | 500 | 10 | 4 | 22,5 à 55,0 | 91,5 |
| 7 | 1 | 5,0/$NH_3$** | 847,5 | 5 | 25,5 | 20 à 43 | 92,9 |

* PHB = p-hydroxybenzaldéhyde.
** On a utilisé de l'ammoniac (gazeux). La réaction se déroule en conditions anhydres.
*** R.M. = rapport molaire d'ammoniac en solution aqueuse de concentration pondérale en pourcent.

*Exemples 8 à 12*

En opérant comme aux exemples 1 à 7, on prépare la vanillylamine (hydroxy-4 méthoxy-3 benzylamine), l'o-vanillylamine (hydroxy-2 méthoxy-3 benzylamine), la salicylamine (hydroxy-2 benzylamine) et l'hydroxy-3 benzylamine, à partir des benzaldéhydes correspondants. On a obtenu les résultats consignés au tableau II.

*Tableau II*

| Ex. N° | Hydroxy-benzal-déhyde | Proportions molaires | | | | Durée (h) | T (° C) | Rdt (%) | Nature des produits obtenus |
|---|---|---|---|---|---|---|---|---|---|
| | | Aldéhyde | $NH_4OH$ (aq.) (R.M./% $NH_3$) | $CH_3OH$ (ml) | Ni humide (g) | | | | |
| 8 | 3-$OCH_3$ 4-OH | 1 | 5/24,2 | 500 | 47 | 3 | 28 à 37 | 96,1 | vanillyl-amine |
| 9 | 2-OH 3-$OCH_3$ | 1 | 5/29,6 | 560 | 26,7 | 5 | 22 à 42 | 93,1 | o-vanillyl-amine |
| 10 | 2-OH | 1 | 5/24,2 | 667 | 26,7 | 7 | 27 à 42 | 92,9 | salicylamine |
| 11 | 2-OH | 1 | 5/$NH_3$ gaz | 583 | 11,7 | 23 | 20 à 42 | 91,5 | salicylamine |
| 12 | 3-OH | 1 | 5/24,2 | 250 | 26,7 | 3 | ambiante | 92,7 | hydroxy-3 benzylamine |

*Exemples 13 à 17*

La p-hydroxybenzylamine obtenue à l'exemple 9 est séparée en cinq portions et l'on fait réagir chaque portion avec du cyanure de sodium en proportion molaire et dans les conditions indiquées au tableau III en effectuant la réaction dans un récipient réactionnel muni d'un agitateur et d'un bain de chauffage à pression atmosphérique. Les résultats obtenus sont présentés au tableau III.

*Tableau III*

| Exemples N° | Proportions molaires | | | Durée (h) | Température (°C) | Rendement (%) |
|---|---|---|---|---|---|---|
| | PHBAM* | NaCN | Solvant (ml/ml) | | | |
| 13 | 1 | 1,26 | DMF (1140) | 8 | 120 | 76,1 |
| 14 | 1 | 1,1 | DMF (1000) | 6 | 120 | 80,5 |
| 15 | 1 | 1,1 | DMF (1082,7) | 3 | 140 | 68,8 |
| 16 | 1 | 1,1 | DMF (570,5) | 3 | 140 | 66,9 |
| 17 | 1 | 1,1 | DMF | 3 | 120 | 68,8 |

* PHBAM = p-hydroxybenzylamine.

*Exemple 18*

On ajoute sous agitation à 1 mol de vanillylamine 1,1 mol de cyanure de sodium et 1 l de diméthylformamide. On chauffe le mélange en agitant à une température de 120°C pendant 15 h. On se sert tout au long de la réaction d'une atmosphère d'argon. Après traitement, on a un rendement de 79,7% en vanillylnitrile distillé.

## Revendications

1. Procédé de préparation de p-hydroxybenzylnitriles à partir du p-hydroxybenzaldéhyde correspondant, en deux étapes, par:

a) réduction d'un benzaldéhyde de formule générale:

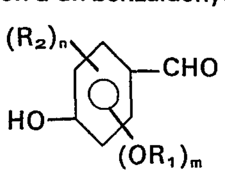

dans laquelle:

– $R_1$ représente un radical méthyle ou éthyle,
– $R_2$ représente un radical méthyle ou éthyle,
– m est un nombre compris entre 0 et 4,
– n est un nombre compris entre 0 et 4,

pour former la benzylamine correspondante de formule générale:

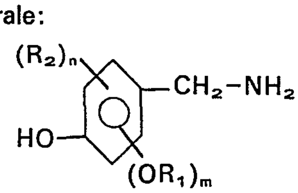

b) remplacement du groupe amino de la benzylamine par un groupe nitrile par réaction avec un cyanure choisi dans le groupe constitué par les cyanures des métaux alcalins et l'acide cyanhydrique, en solution dans un solvant choisi parmi le groupe constitué par le diméthylformamide, le diméthylsulfoxyde et la diméthylpyrrolidone, à une température comprise entre 80°C et la température de reflux de la solution pour former le benzylnitrile correspondant de formule:

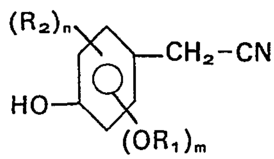

caractérisé en ce que, à l'étape a, la réduction se fait avec de l'hydrogène sous une pression comprise entre la pression atmosphérique et 10 bar, à une température comprise entre 20 et 100°C, dans un milieu réactionnel aqueux comportant de l'ammoniaque en une quantité comprise entre la quantité stœchiométrique et 10 fois la quantité stœchiométrique, et un catalyseur du type nickel de Raney.

2. Procédé selon la revendication 1, caractérisé par le fait que le benzaldéhyde est pris dans le groupe formé par le p-hydroxybenzaldéhyde et le méthoxy-3 hydroxy-4 benzaldéhyde.

3. Procédé selon la revendication 1, caractérisé par le fait que la pression d'hydrogène est comprise entre la pression atmosphérique et 5 bar.

4. Procédé selon la revendication 1, caractérisé par le fait que l'on conduit la réaction dans un récipient clos, pressurisé avec la quantité d'hydrogène requise pour l'amination réductrice, en permettant à la pression dans le récipient d'atteindre le maximum, quel qu'il soit, pour la température de la réaction choisie.

5. Procédé selon la revendication 1, caractérisé en ce que la température de la réaction est la température ambiante.

6. Procédé selon la revendication 1, caractérisé par le fait que la quantité d'ammoniaque est comprise entre 1 et 5 mol par mole de benzaldéhyde.

7. Procédé selon la revendication 1, caractérisé par le fait que la quantité de nickel de Raney utilisé à titre de catalyseur est comprise entre 1 et 25% en poids du mélange réactionnel.

8. Procédé selon la revendication 1, caractérisé par le fait que, lors de l'étape b, la quantité de solvant est comprise entre 300 et 2000 g par mole de benzylamine.

9. Procédé selon la revendication 1, caractérisé par le fait que, lors de l'étape b, on chasse le solvant sous pression réduite et à basse température, que l'on reprend le benzylnitrile dans de l'eau acidulée et que l'on effectue l'extraction du mélange aqueux avec la méthylisobutylcétone ou l'éther isopropylique.

10. Procédé de préparation d'hydroxybenzylamines primaires, caractérisé en ce qu'il consiste essentiellement à réduire un benzaldéhyde présentant la formule:

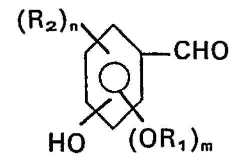

dans laquelle:

le groupe hydroxyle est en position ortho, méta ou para,

$R_1$ représente un radical méthyle ou éthyle,

$R_2$ représente un radical méthyle ou éthyle,

m est un nombre de 0 à 4,

n est un nombre de 0 à 4,

avec de l'hydrogène gazeux sous une pression comprise entre la pression atmosphérique et 10 bar, à une température comprise entre 20 et 100° C, dans un milieu réactionnel aqueux comportant de l'ammoniaque en une quantité comprise entre la stœchiométrie et 10 fois la quantité stœchiométrique et du nickel de Raney à titre de catalyseur, pour former la benzylamine correspondante:

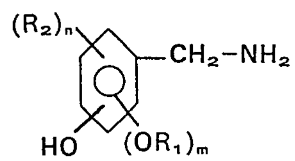

11. Procédé selon la revendication 10, caractérisé par le fait que la pression de l'hydrogène est comprise entre la pression atmosphérique et 5 bar environ.

12. Procédé selon la revendication 10, caractérisé par le fait que l'on conduit la réaction dans un récipient clos, que l'on pressurise avec la quantité d'hydrogène qui est requise pour l'amination réductrice en laissant dans le récipient la pression atteindre un maximum, quel qu'il soit, à la température choisie de la réaction.

13. Procédé selon la revendication 10, caractérisé par le fait que la température réactionnelle est la température ambiante.

14. Procédé selon la revendication 10, caractérisé par le fait que la quantité d'ammoniaque est comprise entre 1 et 5 mol par mole de benzaldéhyde.

15. Procédé selon la revendication 10, caractérisé par le fait que la quantité de nickel de Raney que l'on utilise à titre de catalyseur est comprise entre 1 et 25% du poids du mélange réactionnel.

## Patentansprüche

1. Verfahren zur Herstellung von p-Hydroxybenzylnitrilen aus dem entsprechenden p-Hydroxybenzaldehyd in zwei Stufen durch

a) Reduktion eines Benzaldehyds der allgemeinen Formel

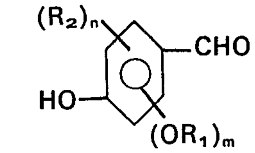

in der

$R_1$ ein Methyl- oder Äthylradikal ist,

$R_2$ ein Methyl- oder Äthylradikal ist,

m eine Zahl zwischen 0 und 4 ist,

n eine Zahl zwischen 0 und 4 ist,

zu dem entsprechenden Benzylamin der allgemeinen Formel

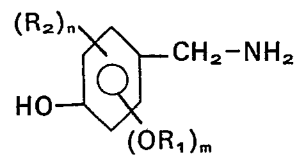

b) Ersatz der Aminogruppe des Benzylamins durch eine Nitrilgruppe durch Reaktion mit einem Cyanid, das aus der Gruppe der Alkalimetallcyanide und der Cyanwasserstoffsäure ausgewählt ist, in Lösung in einem Lösungsmittel, das aus der Gruppe von Dimethylformamid, Dimethylsulfoxid und Dimethylformamid, Dimethylsulfoxid und Dimethylpyrrolidon ausgewählt ist, bei einer Temperatur zwischen 80° C und der Rückflusstemperatur der Lösung zu dem entsprechenden Benzylnitril der Formel

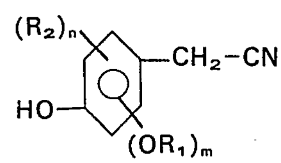

dadurch gekennzeichnet, dass die Reduktion in der Stufe a mit Wasserstoff unter einem Druck zwischen Atmosphärendruck und 10 bar bei einer Temperatur zwischen 20 und 100° C in einer wässerigen Reaktionslösung, die Ammoniak in einer Menge zwischen der stöchiometrischen Menge und dem zehnfachen der stöchiometrischen Menge und einen Katalysator des Raney-Nickel-Typs enthält, durchgeführt wird.

2. Verfahren nach Anspruch 1, dadurch gekennzeichnet, dass der Benzaldehyd aus der Gruppe, die von p-Hydroxybenzaldehyd und 3-Methoxy-4-hydroxybenzaldehyd gebildet wird, gewählt wird.

3. Verfahren nach Anspruch 1, dadurch gekennzeichnet, dass der Wasserstoffdruck zwischen dem atmosphärischen Druck und 5 bar liegt.

4. Verfahren nach Anspruch 1, dadurch gekennzeichnet, dass man die Reaktion in einem geschlossenen Gefäss durchführt, auf das die für die reduktive Aminierung erforderliche Wasserstoffmenge aufgedrückt wird, wobei der Druck im Reaktionsgefäss bis zum maximalen Druck bei der gewählten Reaktionstemperatur ansteigen kann.

5. Verfahren nach Anspruch 1, dadurch gekennzeichnet, dass die Reaktionstemperatur die Umgebungstemperatur ist.

6. Verfahren nach Anspruch 1, dadurch gekennzeichnet, dass die Ammoniakmenge zwischen 1 und 5 mol pro Mol Benzaldehyd liegt.

7. Verfahren nach Anspruch 1, dadurch gekennzeichnet, dass die Menge des als Katalysator eingesetzten Raney-Nickels zwischen 1 und 25 Gew.-% des Reaktionsgemischs beträgt.

8. Verfahren nach Anspruch 1, dadurch gekennzeichnet, dass bei der Stufe b die Menge des Lösungsmittels zwischen 300 und 2000 g pro Mol Benzylamin liegt.

9. Verfahren nach Anspruch 1, dadurch gekennzeichnet, dass bei der Stufe b das Lösungsmittel bei niedriger Temperatur unter vermindertem Druck abgezogen, das Benzylnitril in angesäuertem Wasser aufgenommen und die wässerige Mischung mit Methylisobutylketon oder Isopropyläther extrahiert wird.

10. Verfahren zur Herstellung von primären Hydroxybenzylaminen, dadurch gekennzeichnet, dass es im wesentlichen in der Reduktion eines Benzaldehyds der Formel besteht

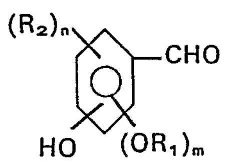

in der die Hydroxygruppe in o-, m- oder p-Stellung ist,
$R_1$ ein Methyl- oder Äthylradikal ist,
$R_2$ ein Methyl- oder Äthylradikal ist,
m eine Zahl von 0 bis 4 ist,
n eine Zahl von 0 bis 4 ist,
mit gasförmigem Wasserstoff unter einem Druck zwischen atmosphärischem Druck und 10 bar bei einer Temperatur zwischen 20 und 100° C in einem wässerigen Reaktionsmedium, das Ammoniak in einer Menge zwischen der stöchiometrischen Menge und dem zehnfachen der stöchiometrischen Menge und Raney-Nickel als Katalysator enthält, zu dem entsprechenden Benzylamin

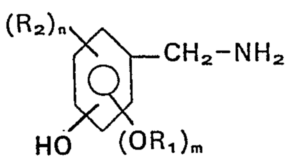

11. Verfahren nach Anspruch 10, dadurch gekennzeichnet, dass der Wasserstoffdruck zwischen atmosphärischem Druck und etwa 5 bar liegt.

12. Verfahren nach Anspruch 10, dadurch gekennzeichnet, dass man die Reaktion in einem geschlossenen Gefäss ausführt, auf das man die zur reduktiven Aminierung erforderliche Wasserstoffmenge aufdrückt, wobei man den Druck in dem Reaktionsgefäss bis zu dem Maximum bei der gewählten Reaktionstemperatur ansteigen lässt.

13. Verfahren nach Anspruch 10, dadurch gekennzeichnet, dass die Reaktionstemperatur die Umgebungstemperatur ist.

14. Verfahren nach Anspruch 10, dadurch gekennzeichnet, dass die Ammoniakmenge zwischen 1 und 5 mol pro Mol Benzaldehyd liegt.

15. Verfahren nach Anspruch 10, dadurch gekennzeichnet, dass die Menge des als Katalysator eingesetzten Raney-Nickels zwischen 1 und 25 Gew.-% des Reaktionsgemischs beträgt.

**Claims**

1. Process for the preparation of p-hydroxybenzylnitriles, from the corresponding p-hydroxybenzaldehyde, in two steps, by

(*a*) reduction of a benzaldehyde of general formula

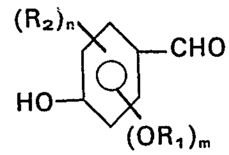

in which
$R_1$ denotes a methyl or ethyl radical,
$R_2$ denotes a methyl or ethyl radical,
m is a number between 0 and 4,
n is a number between 0 and 4,
to form the corresponding benzylamine of general formula

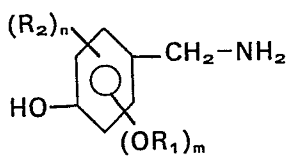

(*b*) the replacement of the amino group of the benzylamine by a nitrile group by reaction with a cyanide chosen from the group consisting of alkali metal cyanides and hydrocyanic acid, in solution in a solvent chosen from the group consisting of dimethylformamide, dimethyl sulphoxide and dimethylpyrrolidone at a temperature of between 80° C and the reflux temperature of the solution to form the corresponding benzylnitrile of formula

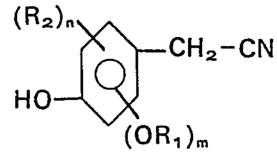

characterized in that in step (*a*) the reduction is carried out with hydrogen under a pressure between atmospheric pressure and 10 bar, at a temperature between 20 and 100° C, in an aqueous reaction medium containing ammonia in a quantity between the stoichiometric quantity and ten times the stoichiometric quantity, and a catalyst of the Raney nickel type.

2. Process according to Claim 1, characterized in that the benzaldehyde is taken from the group consisting of p-hydroxybenzaldehyde and 3-methoxy-4-hydroxybenzaldehyde.

3. Process according to Claim 1, characterized in that the hydrogen pressure is between atmospheric pressure and 5 bar.

4. Process according to Claim 1, characterized in that the reaction is carried out in a closed receptacle pressurised with the quantity of hydrogen required for reductive amination, the pressure in the receptacle being allowed to reach maximum pressure whatever it is, for the chosen reaction temperature.

5. Process according to Claim 1, characterized in that the reaction temperature is the ambient temperature.

6. Process according to Claim 1, characterized in that the quantity of ammonia is between 1 and 5 mol per mole of benzaldehyde.

7. Process according to Claim 1, characterized in that the quantity of Raney nickel employed as catalyst is between 1 and 25% by weight of the reaction mixture.

8. Process according to Claim 1, characterized in that in step (*b*) the quantity of solvent is between 300 and 2,000 g per mole of benzylamine.

9. Process according to Claim 1, characterized in that in step (*b*) the solvent is stripped off under reduced pressure and at low temperature, the benzylnitrile is taken up in acidified water and the extraction of the aqueous mixture is carried out with methyl isobutyl ketone or isopropyl ether.

10. Process for the preparation of primary hydroxybenzylamines, characterized in that it consists substantially in reducing a benzaldehyde having the formula

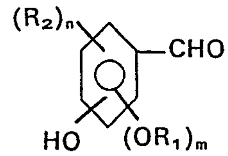

in which the hydroxy group is in ortho, meta or para position,

$R_1$ denotes a methyl or ethyl radical,

$R_2$ denotes a methyl or ethyl radical,

m is a number from 0 to 4,

n is a number from 0 to 4,

with gaseous hydrogen under a pressure between atmospheric pressure and 10 bar, at a temperature between 20 and 100° C, in an aqueous reaction medium containing ammonia in a quantity between the stoichiometry and ten times the stoichiometric quantity and Raney nickel as catalyst, to form the corresponding benzylamine

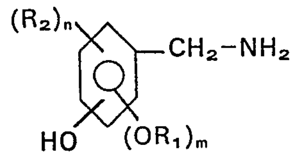

11. Process according to Claim 10, characterized in that the hydrogen pressure is between atmospheric pressure and approximately 5 bar.

12. Process according to Claim 10, characterized in that the reaction is carried out in a closed receptacle, that it is pressurised with the quantity of hydrogen required for reductive amination the pressure in the receptacle being allowed to reach a maximum, whatever it is, at the chosen reaction temperature.

13. Process according to Claim 10, characterized in that the reaction temperature is the ambient temperature.

14. Process according to Claim 10, characterized in that the quantity of ammonia is between 1 and 5 mol per mole of benzaldehyde.

15. Process according to Claim 10, characterized in that the quantity of Raney nickel employed as catalyst is between 1 and 25% by weight of the reaction mixture.